# EUROPEAN PATENT APPLICATION

(11) **EP 4 464 702 A1**
(43) Date of publication of application: **20.11.2024**
(21) Application number: 23740303.5
(22) Date of filing: 12.01.2023
(51) Int. Cl.: C07D 413/14, C07D 417/14, H05B 33/02, H10K 50/00, H10K 50/15, H10K 50/16

(54) **PYRIMIDINE COMPOUND AND ORGANIC ELECTROLUMINESCENT ELEMENT**

(30) Priority: 13.01.2022 JP 2022003494
(71) Applicant: Hodogaya Chemical Co., Ltd., Tokyo, 105-0021 (JP)
(72) Inventor: KASE, Kouki, Tokyo 105-0021 (JP); HIRAI, Hiroki, Tokyo 105-0021 (JP); HWANG, Moon-Chan, Tokyo 105-0021 (JP); HIRAYAMA, Yuta, Tokyo 105-0021 (JP); HAYASHI, Shuichi, Tokyo 105-0021 (JP)
(74) Representative: Forstmeyer, Dietmar
(86) International application number: PCT/JP2023/000615
(87) International publication number: WO 2023/136295

(57) **Abstract**

It is an object of the present invention to provide a compound that has a high refractive index and a low extinction coefficient in a wavelength range of 450 to 750 nm, and is thus suitably used as a material for a capping layer of an organic EL element. Also, it is another object of the invention to provide an organic EL element including such a compound to improve the light extraction efficiency of the element. The invention is directed to a pyrimidine compound represented by a general formula (1): 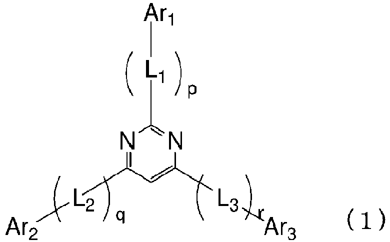 wherein Ar₁ to Ar₃ are the same or different, and each represent a substituted or unsubstituted aromatic hydrocarbon group, a substituted or unsubstituted aromatic heterocyclic group, or the like, L₁ to L₃ are the same or different, and each represent a single bond, a substituted or unsubstituted divalent aromatic hydrocarbon group, or the like, p, q, and r are the same or different, and each represent an integer of 1 or 2, and at least one of Ar₁ to Ar₃ is a substituted or unsubstituted benzoxazolyl or other aromatic heterocyclic group.

## Description

### Technical Field

The present invention relates to a compound suitable for a self-emissive electronic element favorably used in various display devices, and particularly a compound suitable for an organic electroluminescent element (hereinafter abbreviated as an "organic EL element"), and also relates to an organic EL element, an electronic element, and an electronic device including the compound.

### Background Art

Since organic EL elements are self-emissive elements, they have greater brightness and better viewability than elements including liquid crystals, and can thus provide a clearer display. For these reasons, active studies have been carried out on organic EL elements.

In 1987, C. W. Tang et al. of Eastman Kodak Company developed an element having a layered structure in which various functions were assigned to different materials, and thus made a practical organic EL element including organic materials. They made an organic EL element having a layered structure including a layer of a fluorescent substance capable of transporting electrons and a layer of an organic substance capable of transporting holes, and injected both charges into the layer of the fluorescent substance to thereby cause light emission, and the organic EL element thus achieved a luminance as high as 1,000 cd/m² or more at a voltage of 10 V or less (see Patent Literatures 1 and 2, for example).

Organic EL elements have been heretofore much improved to put them to practical use. Electroluminescent elements with a bottom emission structure, which emit light from the bottom thereof, have been suggested in which an anode, a hole-injecting layer, a hole-transporting layer, a light-emitting layer, an electron-transporting layer, an electron-injecting layer, and a cathode are sequentially provided on a substrate to subdivide various functions in the multi-layered structure even further. Such electroluminescent elements successfully have high efficiency and durability (see Non-Patent Literature 1, for example).

Recently, light-emitting elements with a top emission structure are coming into use. In the light-emitting element with a top emission structure, a metal having a high work function is used as an anode, and light is extracted from the top side of the element. A light-emitting element with a bottom emission structure results in a limited area of the light-emitting portion, since light is extracted from its bottom side, which has circuits for pixels. In contrast, the light-emitting element with a top emission structure advantageously enables a large light-emitting area since light extracted from the top side is not blocked by the circuits for pixels. In light-emitting elements with a top emission structure, translucent electrodes made of LiF/Al/Ag (see Non-Patent Literature 2, for example), Ca/Mg (see Non-Patent Literature 3, for example), LiF/MgAg, or the like are used for a cathode.

In such light-emitting elements, when light is emitted by a light-emitting layer and enters into another film, the light is totally reflected at an interface between the light-emitting layer and the other film, if the light enters at a certain angle or larger. Accordingly, it is only a part of the emitted light that is available. Recently, light-emitting elements have been proposed in which a "capping layer" with a high refractive index is provided on the outside of a translucent electrode with a low refractive index, to improve the light extraction efficiency (see Non-Patent Literatures 2 and 3, for example).

The effect of the capping layer in a light-emitting element with a top emission structure including Ir(ppy)₃ as a light-emitting material was as follows: while a light-emitting element without a capping layer has a current efficiency of 38 cd/A, a light-emitting element including a ZnSe film with a thickness of 60 nm as a capping layer has a current efficiency of 64 cd/A, which indicates that the efficiency is improved about 1.7 times. Furthermore, it has been shown that the maximum point of the transmittance of the translucent electrode and the capping layer and the maximum point of the efficiency do not necessarily match each other, and it is thus indicated that the maximum point of the light extraction efficiency depends on the interference effects (see Non-Patent Literature 3, for example)..

Conventionally, it has been proposed to use a fine metal mask to form a capping layer, but the metal mask is disadvantageously deformed by heat when used at a high temperature, which deteriorates the positioning precision. For example, ZnSe has a melting point as high as 1100°C or higher (see Non-Patent Literature 3, for example), and thus cannot be vapor-deposited at an accurate position with a fine metal mask, which may affect the light-emitting element. Furthermore, film formation by sputtering also affects the light-emitting element. Thus a capping layer made of an inorganic material as a constituent material cannot be suitably used.

In addition, it has been proposed to use tris(8-hydroxyquinoline)aluminum (hereinafter abbreviated as "Alq₃") for a capping layer for adjusting the refractive index (see Non-Patent Literature 2, for example). However, Alq₃ is known as an organic EL material commonly used as a green light-emitting material or electron-transporting material, and exhibits small absorption at about 450 nm, which is near the wavelength of light emitted by blue light-emitting materials. Thus, when Alq₃ is used for blue light-emitting elements, problems of a poor color purity and a small efficiency in light extraction arise.

In view of improving the characteristics of an organic EL element and also significantly improving the light extraction efficiency thereof, there is a demand for a material for a capping layer that has a high refractive index and a low extinction coefficient and also has excellent stability and durability in the form of a thin film.

### Citation List

### Patent Literatures

Patent Literature 1: US 5792557
Patent Literature 2: US 5639914
Patent Literature 3: WO 2014/009310
Patent Literature 4: US 2014/0225100A1

### Non-Patent Literatures

Non-Patent Literature 1: Proceedings of the 9th Meeting of the Japan Society of Applied Physics, pp. 55-61 (2001)
Non-Patent Literature 2: Appl. Phys. Let., 78, 544 (2001)
Non-Patent Literature 3: Appl. Phys. Let., 82, 466 (2003)
Non-Patent Literature 4: Tetrahedron, 58, 9633 (2002)
Non-Patent Literature 5: Appl. Phys. Let., 98, 083302 (2011)

### Summary of Invention

It is an object of the present invention to provide a compound that has a high refractive index and a low extinction coefficient in a wavelength range of 450 to 750 nm, and is thus suitably used as a material for a capping layer of an organic EL element. It is another object of the invention to provide an organic EL element including such a compound to improve the light extraction efficiency of the element.

A compound suitably used for a capping layer of an organic EL element should have the following physical properties, for example: (1) a high refractive index, (2) a low extinction coefficient, (3) vapor-depositability, (4) good stability in the form of a thin film, and (5) a high glass transition point. The present invention intends to provide an organic EL element having the following physical properties, for example: (1) high light extraction efficiency, (2) no deterioration in the color purity, (3) the ability to transmit light without change over time, and (4) a long life.

To achieve the above-described objects, the inventors have focused on the fact that compounds having a pyrimidine skeleton are excellent in stability and also durability when it is in the form of a thin film, and have optimized the molecular design thereof to develop a material that has a high refractive index and a low extinction coefficient in a wavelength range of 450 to 750 nm. Furthermore, the inventors have produced an organic EL element including the resulting compound and thoroughly evaluated the properties thereof, and as a result, the conventional problems have been solved, thus accomplishing the present invention.

Specifically, the present invention is directed to a pyrimidine compound represented by a general formula (1) below and an organic EL element.

1) A pyrimidine compound represented by a general formula (1) below. 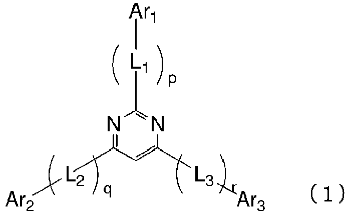
   wherein Ar₁ to Ar₃ are the same or different, and each represent a substituted or unsubstituted aromatic hydrocarbon group, a substituted or unsubstituted aromatic heterocyclic group, or a substituted or unsubstituted fused polycyclic aromatic group,
   L₁ to L₃ are the same or different, and each represent a single bond, a substituted or unsubstituted divalent aromatic hydrocarbon group, a substituted or unsubstituted divalent aromatic heterocyclic group, or a substituted or unsubstituted divalent fused polycyclic aromatic group, and
   p, q, and r are the same or different, and each represent an integer of 1 or 2, and
   at least one of Ar₁ to Ar₃ is a substituted or unsubstituted benzoxazolyl group, a substituted or unsubstituted benzothiazolyl group, a substituted or unsubstituted benzofuranyl group, or a substituted or unsubstituted benzothienyl group.
2) The pyrimidine compound as set forth in 1), wherein L₁ to L₃ in the general formula (1) above are the same or different, and each represent a single bond, a divalent group obtained by reducing two hydrogen atoms from a substituted or unsubstituted benzene, a divalent group obtained by reducing two hydrogen atoms from a substituted or unsubstituted biphenyl, or a divalent group obtained by reducing two hydrogen atoms from a substituted or unsubstituted naphthalene.
3) The pyrimidine compound as set forth in 2), wherein L₁ to L₃ in the general formula (1) above are the same or different, and each represent a single bond, a divalent group obtained by reducing two hydrogen atoms from an unsubstituted benzene, a divalent group obtained by reducing two hydrogen atoms from an unsubstituted biphenyl, or a divalent group obtained by reducing two hydrogen atoms from an unsubstituted naphthalene.
4) The pyrimidine compound as set forth in 3), wherein L₁ to L₃ in the general formula (1) above are the same or different, and each represent a single bond, an unsubstituted 1,4-phenylene group, an unsubstituted 4,4'-biphenylylene group, an unsubstituted 2,6-naphthylene group, or an unsubstituted 2,7-naphthylene group.
5) The pyrimidine compound as set forth in any one of 1) to 4), wherein p, q, and r in the general formula (1) above are 1.
6) The pyrimidine compound as set forth in any one of 1) to 5), wherein at least one of Ar₁ to Ar₃ in the general formula (1) above is an unsubstituted benzoxazolyl group, an unsubstituted benzothiazolyl group, an unsubstituted benzofuranyl group, or an unsubstituted benzothienyl group.
7) The pyrimidine compound as set forth in 6), wherein at least one of Ar₁ to Ar₃ in the general formula (1) above is an unsubstituted 2-benzoxazolyl group, an unsubstituted 2-benzothiazolyl group, an unsubstituted 2-benzofuranyl group, or an unsubstituted 2-benzothienyl group.
8) The pyrimidine compound as set forth in 7), wherein any two of Ar₁ to Ar₃ in the general formula (1) above are an unsubstituted 2-benzoxazolyl group, an unsubstituted 2-benzothiazolyl group, an unsubstituted 2-benzofuranyl group, or an unsubstituted 2-benzothienyl group.
9) The pyrimidine compound as set forth in 7), wherein Ar₁ to Ar₃ in the general formula (1) above are an unsubstituted 2-benzoxazolyl group, an unsubstituted 2-benzothiazolyl group, an unsubstituted 2-benzofuranyl group, or an unsubstituted 2-benzothienyl group.
10) The pyrimidine compound as set forth in any one of 1) to 9), having a refractive index of 1.70 or more, in a wavelength range of 450 to 750 nm.
11) An organic EL element including, at least, an anode, a hole-transporting layer, a light-emitting layer, an electron-transporting layer, a cathode, and a capping layer arranged in this order, wherein the capping layer contains the pyrimidine compound as set forth in any one of 1) to 10).
12) An organic EL element, wherein the capping layer is a mixed layer containing two or more compounds or a stack of two or more layers containing respective compounds different from each other, and at least one of the compounds contains the pyrimidine compound as set forth in any one of 1) to 10).
13) An electronic element including a pair of electrodes and at least one organic layer interposed therebetween, wherein the organic layer contains the pyrimidine compound as set forth in any one of 1) to 10).
14) An electronic device including the electronic element as set forth in 13).

The "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by Ar₁ to Ar₃ in the general formula (1) may specifically refer to a group selected from aryl groups having 6 to 30 carbon atoms and heteroaryl groups having 2 to 20 carbon atoms, such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a quinazolinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

The "divalent aromatic hydrocarbon group", the "divalent aromatic heterocyclic group", or the "divalent fused polycyclic aromatic group" in the "substituted or unsubstituted divalent aromatic hydrocarbon group", the "substituted or unsubstituted divalent aromatic heterocyclic group", or the "substituted or unsubstituted divalent fused polycyclic aromatic group" represented by L₁ to L₃ in the general formula (1) may refer to a divalent group obtained by reducing one hydrogen atom from the "aromatic hydrocarbon group", the "aromatic heterocyclic group", or the "fused polycyclic aromatic group" listed above as those represented by Ar₁ to Ar₃ in the general formula (1).

The "substituent" in the "substituted aromatic hydrocarbon group", the "substituted aromatic heterocyclic group", or the "substituted fused polycyclic aromatic group" represented by Ar₁ to Ar₃ and L₁ to L₃ in the general formula (1) may specifically refer to: a heavy hydrogen atom, a cyano group, or a nitro group; a halogen atom such as a fluorine atom, a chlorine atom, a bromine atom, or an iodine atom; a silyl group such as a trimethylsilyl group or a triphenylsilyl group; a linear or branched alkyl group having 1 to 6 carbon atoms such as a methyl group, an ethyl group, or a propyl group; a linear or branched alkyloxy group having 1 to 6 carbon atoms such as a methyloxy group, an ethyloxy group, or a propyloxy group; an alkenyl group such as a vinyl group or an allyl group; an aryloxy group such as a phenyloxy group or a tolyloxy group; an arylalkyloxy group such as a benzyloxy group or a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, or a triphenylenyl group; a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a quinazolinyl group, a benzimidazolyl group, a pyrazolyl group, a dibenzofuranyl group, a dibenzothienyl group, or a carbolinyl group, or may refer to an aryl group having 6 to 30 carbon atoms or a heteroaryl group having 2 to 20 carbon atoms. These substituents may be further substituted with the above-listed substituents. Furthermore, a substituent and the benzene ring substituted therewith, or a plurality of substituents on the same benzene ring may be bonded to each other via a single bond, a substituted or unsubstituted methylene group, an oxygen atom, or a sulfur atom to form a ring.

Preferably, at least two of Ar₁ to Ar₃ in the general formula (1) are a substituted or unsubstituted benzoxazole group, a substituted or unsubstituted benzothiazole group, a substituted or unsubstituted benzofuranyl group, or a substituted or unsubstituted benzothienyl group. More preferably, all of Ar₁ to Ar₃ are a substituted or unsubstituted benzoxazole group, a substituted or unsubstituted benzothiazole group, a substituted or unsubstituted benzofuranyl group, or a substituted or unsubstituted benzothienyl group.

At least one of Ar₁ to Ar₃ in the general formula (1) is preferably an unsubstituted benzoxazolyl group, an unsubstituted benzothiazolyl group, an unsubstituted benzofuranyl group, or an unsubstituted benzothienyl group, and more preferably an unsubstituted 2-benzoxazolyl group, an unsubstituted 2-benzothiazolyl group, an unsubstituted 2-benzofuranyl group, or an unsubstituted 2-benzothienyl group.

It is preferable that any two of Ar₁ to Ar₃ in the general formula (1) should be an unsubstituted 2-benzoxazolyl group, an unsubstituted 2-benzothiazolyl group, an unsubstituted 2-benzofuranyl group, or an unsubstituted 2-benzothienyl group. It is also preferable that all of Ar₁ to Ar₃ should be an unsubstituted 2-benzoxazolyl group, an unsubstituted 2-benzothiazolyl group, an unsubstituted 2-benzofuranyl group, or an unsubstituted 2-benzothienyl group.

L₁ to L₃ in the general formula (1) are the same or different, and preferably represent a single bond, a divalent group obtained by reducing two hydrogen atoms from an unsubstituted benzene, a divalent group obtained by reducing two hydrogen atoms from an unsubstituted biphenyl, or a divalent group obtained by reducing two hydrogen atoms from an unsubstituted naphthalene, and more preferably represent a single bond, a divalent group obtained by reducing two hydrogen atoms from an unsubstituted benzene, a divalent group obtained by reducing two hydrogen atoms from an unsubstituted biphenyl, or a divalent group obtained by reducing two hydrogen atoms from an unsubstituted naphthalene.

L₁ to L₃ in the general formula (1) are the same or different, and preferably represent a single bond, an unsubstituted 1,4-phenylene group, an unsubstituted 4,4'-biphenylylene group, an unsubstituted 2,6-naphthylene group, or an unsubstituted 2,7-naphthylene group, more preferably represent a single bond, an unsubstituted 1,4-phenylene group, or an unsubstituted 4,4'-biphenylylene group, and even more preferably a single bond or an unsubstituted 1,4-phenylene group.

All of p, q, and r in the general formula (1) are preferably 1.

In the organic EL element, the capping layer has a thickness of preferably 30 to 120 nm, and more preferably 40 to 80 nm.

The compound represented by the general formula (1) above of the present invention has (1) a high refractive index in a wavelength range of 450 to 750 nm, (2) a low extinction coefficient, (3) vapor-depositability, (4) good stability in the form of a thin film, and (5) a high thermal resistance. Accordingly, light extraction efficiency is significantly improved by providing a capping layer containing such a compound on the outside of a transparent or translucent electrode of an organic EL element.

### Brief Description of Drawings

[Fig. 1] The figure shows the structures of Compounds (1-1) to (1-12) as examples of the compound of the present invention.
[Fig. 2] The figure shows the structures of Compounds (1-13) to (1-24) as examples of the compound of the present invention.
[Fig. 3] The figure shows the structures of Compounds (1-25) to (1-36) as examples of the compound of the present invention.
[Fig. 4] The figure shows the structures of Compounds (1-37) to (1-48) as examples of the compound of the present invention.
[Fig. 5] The figure shows the structures of Compounds (1-49) to (1-60) as examples of the compound of the present invention.
[Fig. 6] The figure shows the structures of Compounds (1-61) to (1-72) as examples of the compound of the present invention.
[Fig. 7] The figure shows the structures of Compounds (1-73) to (1-84) as examples of the compound of the present invention.
[Fig. 8] The figure shows the structures of Compounds (1-85) to (1-96) as examples of the compound of the present invention.
[Fig. 9] The figure shows the structures of Compounds (1-97) to (1-108) as examples of the compound of the present invention.
[Fig. 10] The figure shows the structures of Compounds (1-109) to (1-120) as examples of the compound of the present invention.
[Fig. 11] The figure shows the structures of Compounds (1-121) to (1-132) as examples of the compound of the present invention.
[Fig. 12]The figure shows the structures of Compounds (1-133) to (1-144) as examples of the compound of the present invention.
[Fig. 13]The figure shows the structures of Compounds (1-145) to (1-156) as examples of the compound of the present invention.
[Fig. 14]The figure shows the structures of Compounds (1-157) to (1-168) as examples of the compound of the present invention.
[Fig. 15]The figure shows the structures of Compounds (1-169) to (1-180) as examples of the compound of the present invention.
[Fig. 16]The figure shows the structures of Compounds (1-181) to (1-189) as examples of the compound of the present invention.
[Fig. 17]The figure shows the structures of Compounds (1-190) to (1-197) as examples of the compound of the present invention.
[Fig. 18] The figure shows an example of the structure of the organic EL element of the present invention.

### Description of Embodiment

The pyrimidine compound represented by the general formula (1) above is a novel compound, but can be synthesized, for example, by a known coupling reaction with a palladium catalyst or the like (see Non-Patent Literature 4, for example).

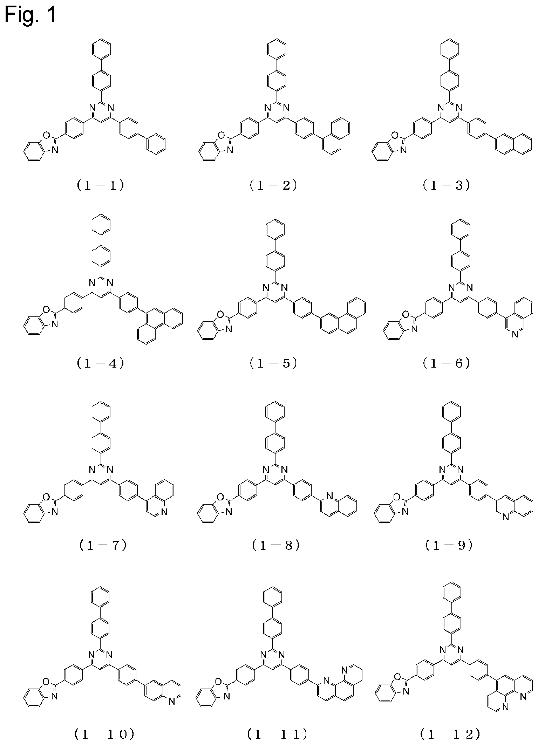

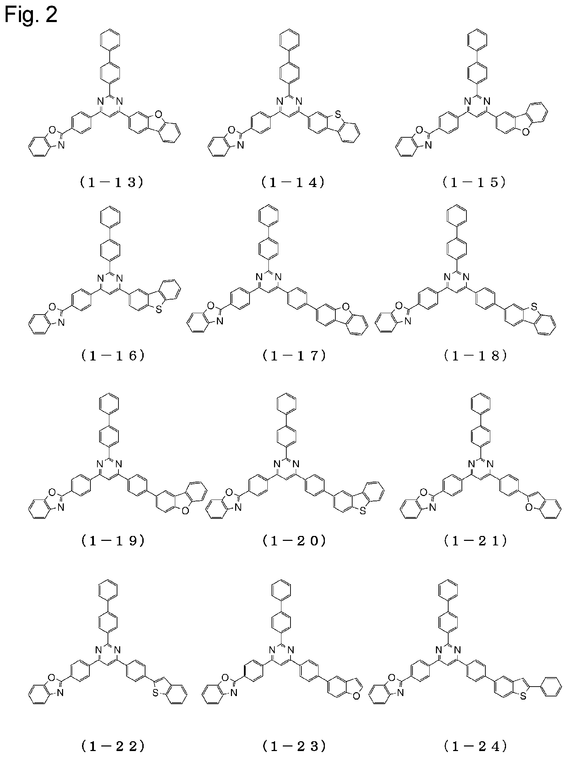

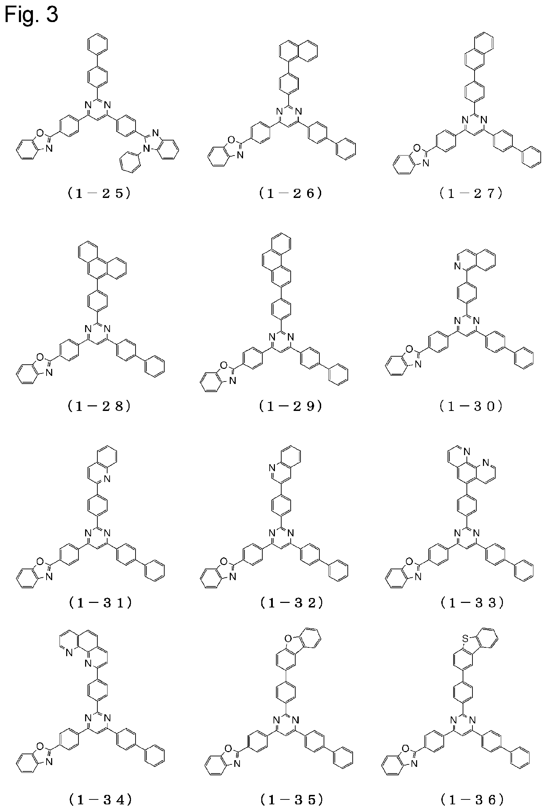

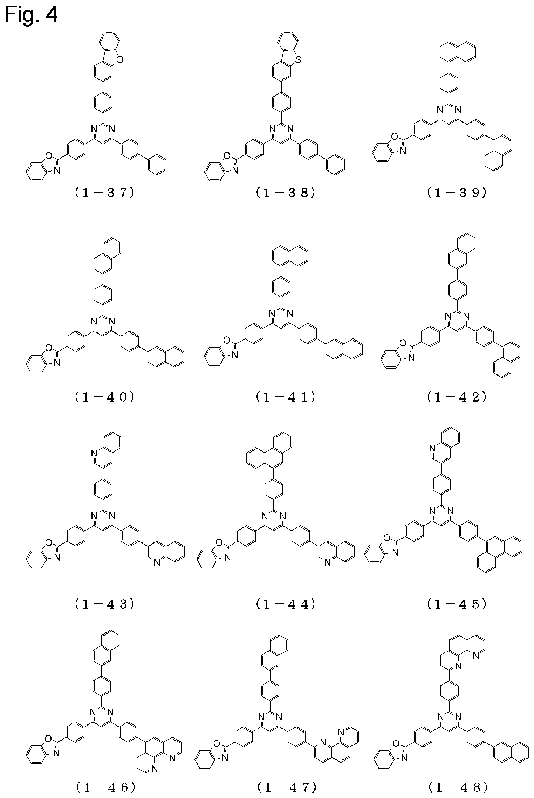

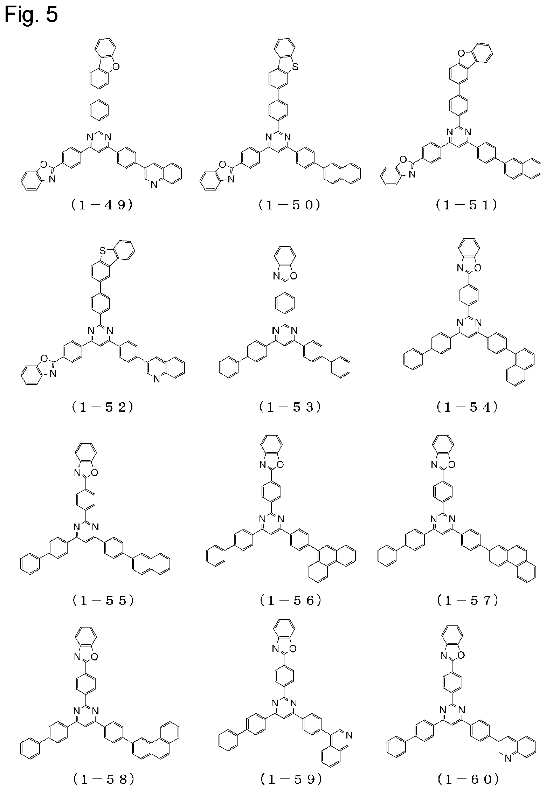

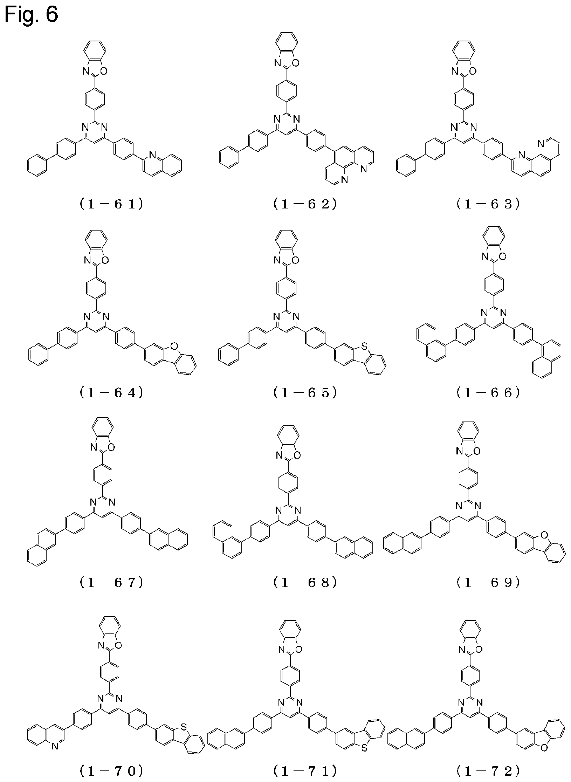

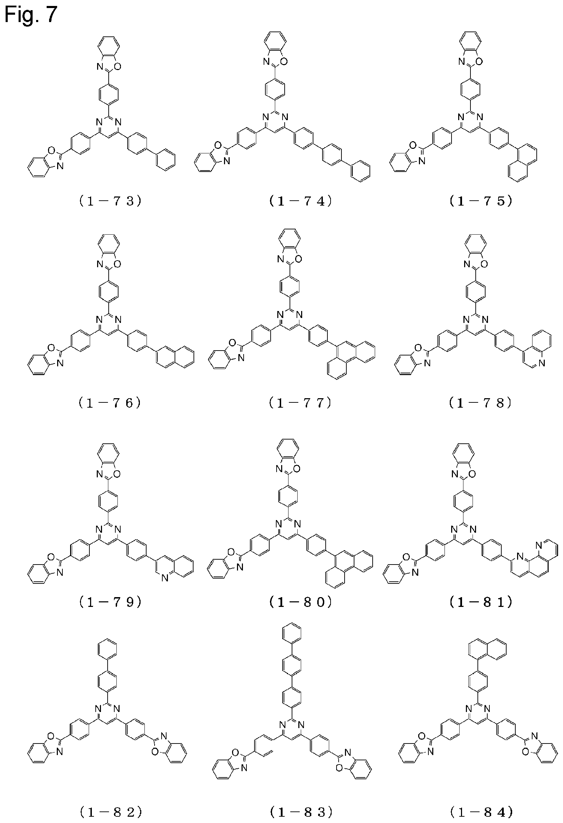

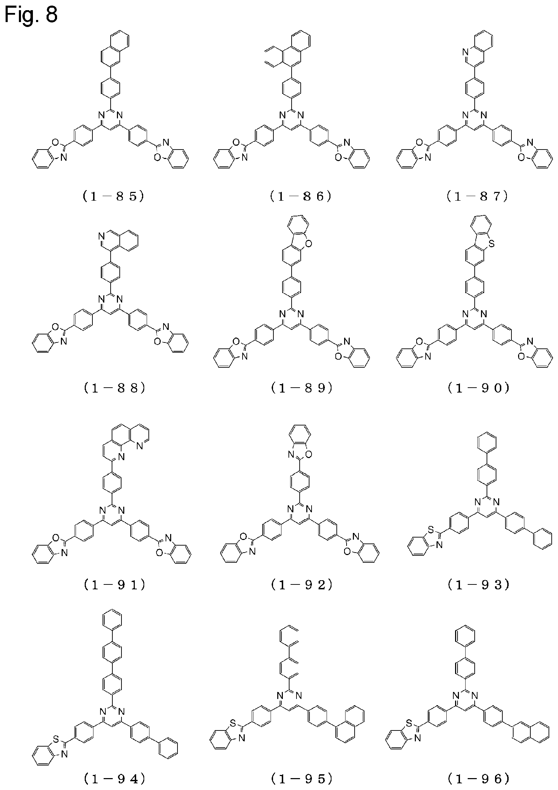

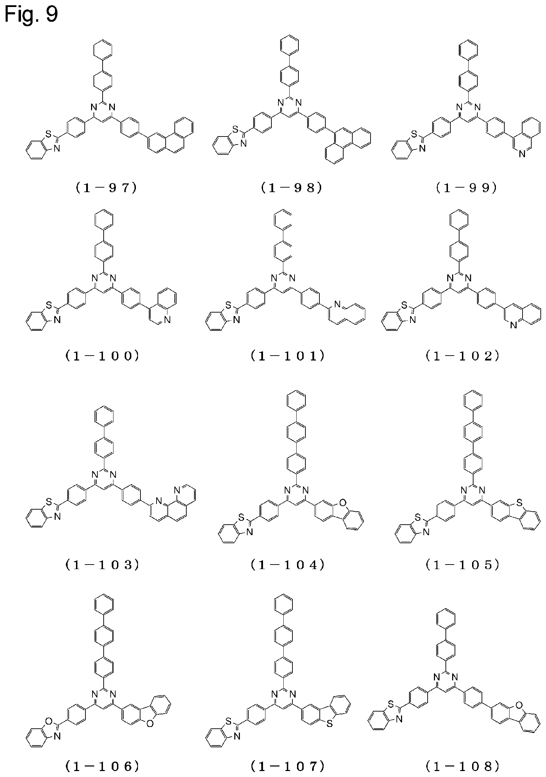

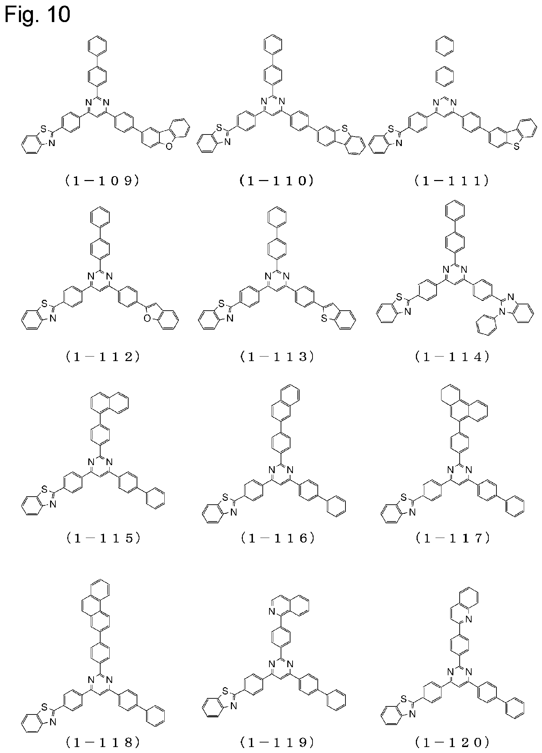

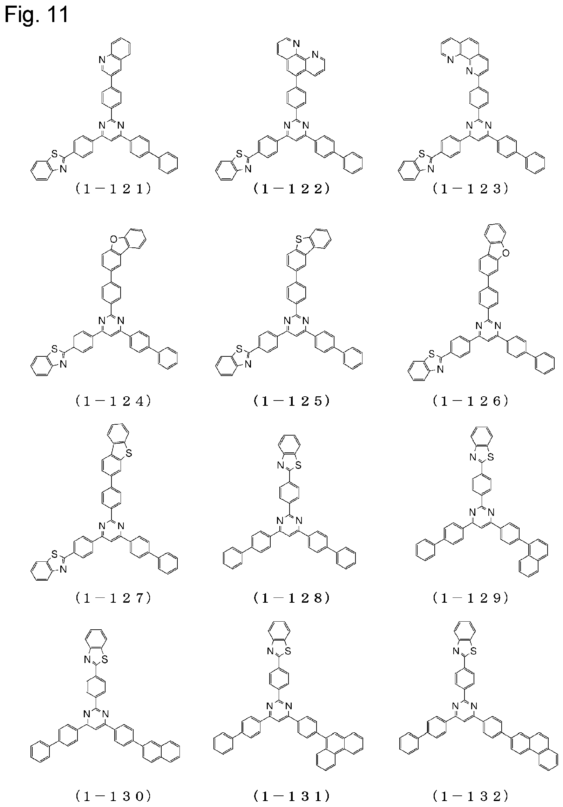

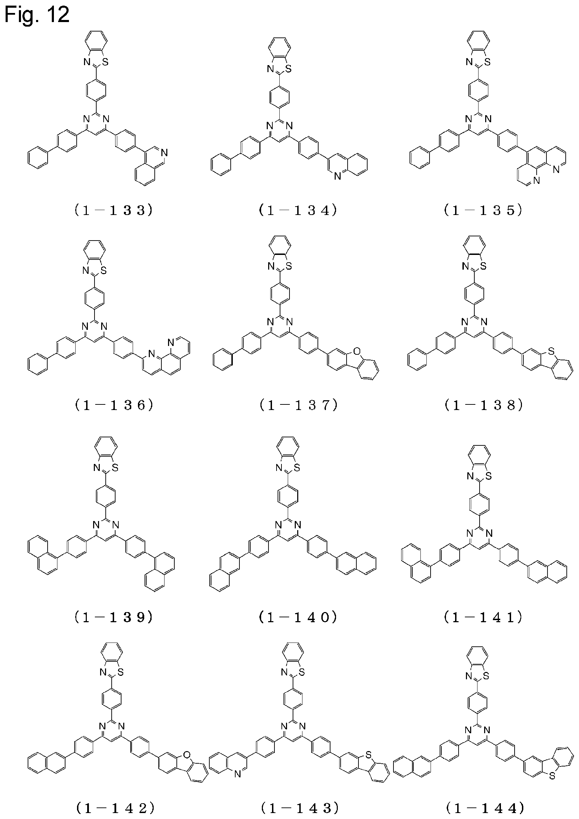

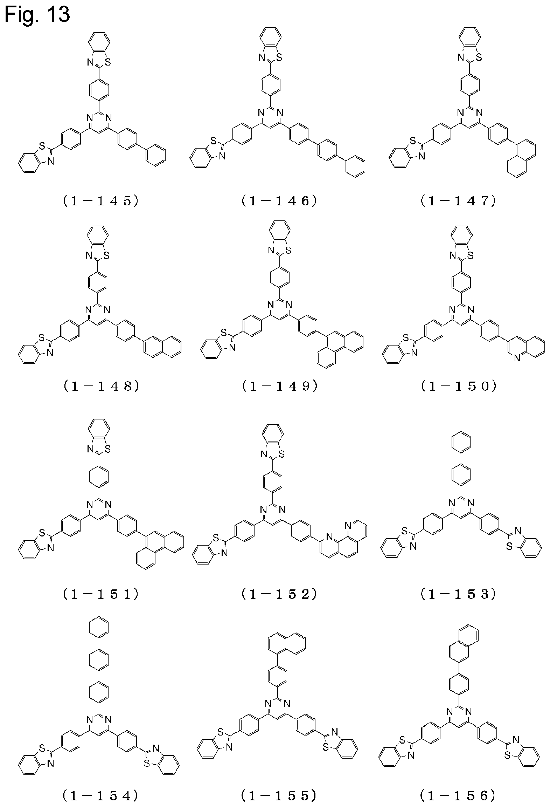

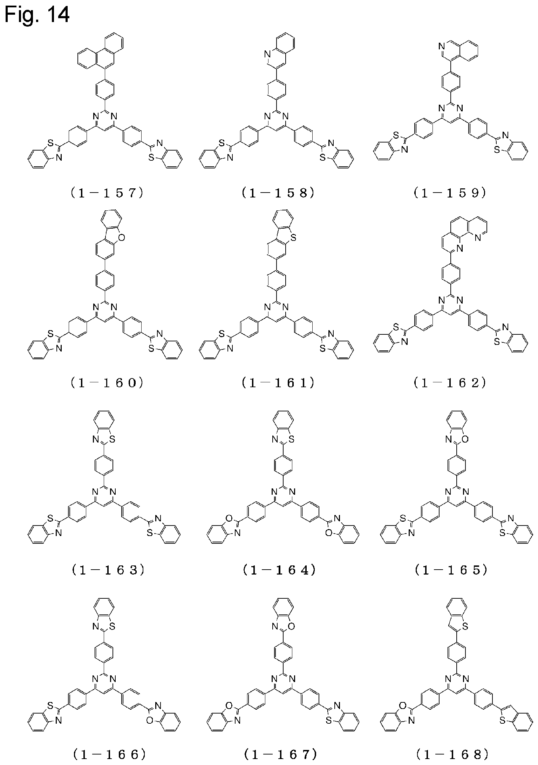

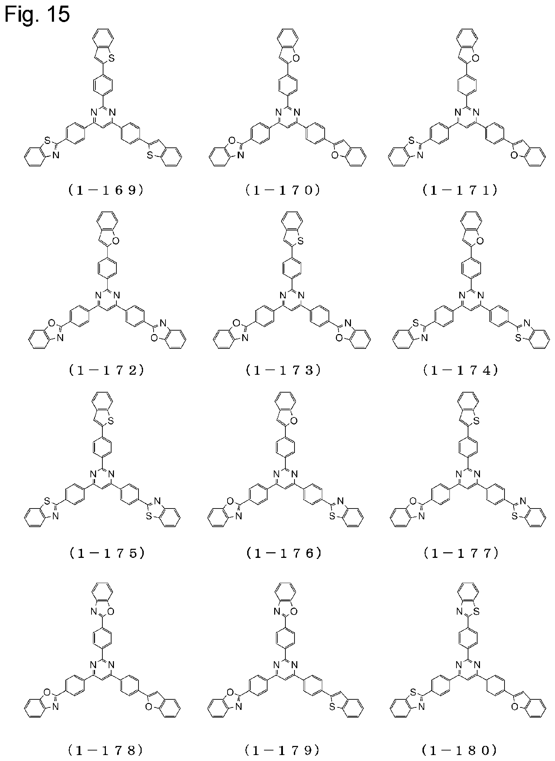

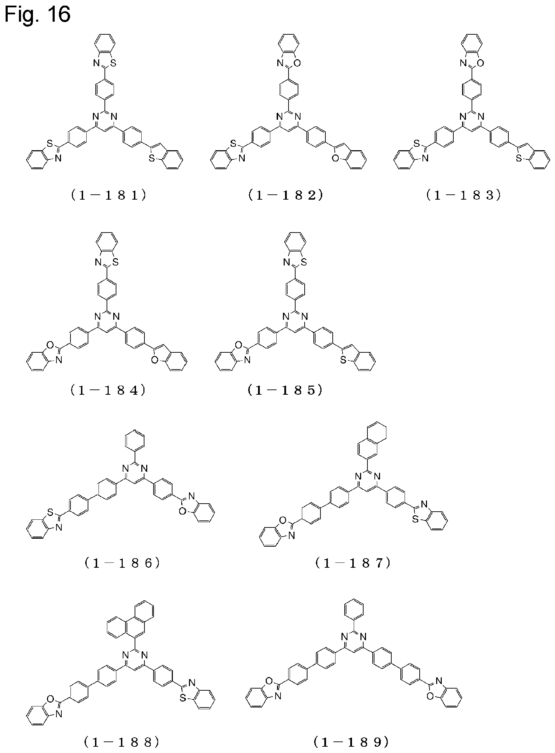

Figs. 1 to 17 show specific examples of preferred pyrimidine compounds among those represented by the general formula (1) above, but the invention is not limited to these compounds.

There is no particular limitation on the method for purifying the compound represented by the general formula (1) above, and any known methods for purification of organic compounds can be used, including column chromatography, adsorption using silica gel, activated carbon, activated clay, or the like, recrystallization and crystallization from a solvent, and sublimation. The compound can be identified by NMR analysis. The melting point, the glass transition point (Tg), the refractive index, and the extinction coefficient are preferably measured as physical properties. The melting point is a measure of vapor deposition properties, the glass transition point (Tg) is a measure of the stability in the form of a thin film, and the refractive index and extinction coefficient are measures of the improvement of light extraction efficiency.

The melting point and the glass transition point (Tg) can be measured on a powder using a high-sensitivity differential scanning calorimeter (DSC3100SA manufactured by Bruker AXS K.K.).

The refractive index and the extinction coefficient can be measured on an 80-nm thin film formed on a silicon substrate, using a spectroscopic analyzer (F10-RT-UV manufactured by Filmetrics).

The organic EL element for use as a light emitting element with a top emission structure may have a structure in which an anode, a hole-transporting layer, a light-emitting layer, an electron-transporting layer, a cathode, and a capping layer are sequentially provided on a glass substrate, for example. The structure may further include any of a hole-injecting layer between the anode and the hole-transporting layer; an electron-blocking layer between the hole-transporting layer and the light-emitting layer; a hole-blocking layer between the light-emitting layer and the electron-transporting layer; and an electron-injecting layer between the electron-transporting layer and the cathode. In these multilayer structures, a single organic layer may perform the functions of some layers. For example, a single organic layer may serve as both the hole-injecting layer and the hole-transporting layer; both the hole-transporting layer and the electron-blocking layer; both the hole-blocking layer and the electron-transporting layer; or both the electron-transporting layer and the electron-injecting layer. It is also possible to stack two or more organic layers having the same function. Specifically, two hole-transporting layers may be stacked; two light-emitting layers may be stacked; two electron-transporting layers may be stacked; and two capping layers may be stacked, for example.

The total film thickness of the layers of the organic EL element is preferably from 200 to 750 nm, and more preferably from 350 to 600 nm. For example, the film thickness of the capping layer is preferably from 30 to 120 nm, and more preferably from 40 to 80 nm. This results in good light extraction efficiency. The film thickness of the capping layer may be tailored to the type of light-emitting material used for the light-emitting element, the thickness of the organic EL element excluding the capping layer, and others.

An electrode material having a high work function, such as ITO or gold, is used for the anode of the organic EL element.

Examples of a material for the hole-injecting layer of the organic EL element include arylamine compounds having a molecular structure in which three or more triphenylamine structures are bonded to each other via a single bond or a hetero atom-free divalent group, such as starburst triphenylamine derivatives, and various triphenylamine tetramers; porphyrin compounds typified by copper phthalocyanine; acceptor type heterocyclic compounds such as hexacyanoazatriphenylene; and coating type polymer materials. These materials may be used singly to form a film as the layer, or two or more thereof may be mixed and used to form a single layer. Alternatively, these materials may be used to form a layered structure, and the layered structure may be composed of different layers each formed of a single kind of material, or composed of different layers each formed of a mixture of materials, or composed of a layer formed of a single kind of material and a layer formed of a mixture of materials. The material or materials can be formed into a thin film by a known method, such as vapor deposition, spin coating or inkjet printing.

Examples of as a material used for the hole-transporting layer of the organic EL element include benzidine derivatives such as N,N'-diphenyl-N,N'-di(m-tolyl)benzidine (hereinafter abbreviated as "TPD"), N,N'-diphenyl-N,N'-di(α-naphthyl)benzidine, and N,N,N',N'-tetrabiphenylyl benzidine, and 1,1-bis[4-(di-4-tolylamino)phenyl]cyclohexane. In particular, it is preferable to use an arylamine compound having a molecular structure in which two triphenylamine structures are bonded to each other via a single bond or a hetero atom-free divalent group, including N,N,N',N'-tetrabiphenylyl benzidine. It is also preferable to use an arylamine compound having a molecular structure in which three or more triphenylamine structures are bonded to each other via a single bond or a hetero atom-free divalent group, including various triphenylamine trimers and tetramers. These materials may be used singly to form a film as the layer, or two or more thereof may be mixed and used to form a single layer. Alternatively, these materials may be used to form a layered structure, and the layered structure may be composed of different layers each formed of a single kind of material, or composed of different layers each formed of a mixture of materials, or composed of a layer formed of a single kind of material and a layer formed of a mixture of materials. Furthermore, coating type polymer materials such as poly(3,4-ethylenedioxythiophene)/poly(styrenesulfonate) can be used for the hole-injecting/transporting layer. The material or materials can be formed into a thin film by a known method, such as vapor deposition, spin coating or inkjet printing.

Furthermore, other examples of a material used for the hole-injecting layer or the hole-transporting layer include those obtained by p-doping a material normally used for these layers with a dopant such as trisbromophenylamine hexachloroantimony or a radialene derivative (see Patent Literature 3, for example), and a polymer compound having the structure of a benzidine derivative, such as TPD, as a partial structure thereof.

Furthermore, the organic EL element can include an electron-blocking layer. Examples of a material used for the electron-blocking layer include compounds having an electron blocking effect, such as carbazole derivatives such as 4,4',4"-tri(N-carbazolyl)triphenylamine (hereinafter abbreviated as "TCTA"), 9,9-bis[4-(carbazole-9-yl)phenyl]fluorene, 1,3-bis(carbazole-9-yl)benzene (hereinafter abbreviated as "mCP"), and 2,2-bis(4-carbazole-9-ylphenyl)adamantane; and compounds having a triphenylsilyl group and a triarylamine structure and typified by 9-[4-(carbazole-9-yl)phenyl]-9-[4-(triphenylsilyl)phenyl]-9H-fluorene. These materials may be used singly to form a film as the layer, or two or more thereof may be mixed and used to form a single layer. Alternatively, these materials may be used to form a layered structure, and the layered structure may be composed of different layers each formed of a single kind of material, or composed of different layers each formed of a mixture of materials, or composed of a layer formed of a single kind of material and a layer formed of a mixture of materials. The material or materials can be formed into a thin film by a known method, such as vapor deposition, spin coating or inkjet printing.

Examples of a material used for the light-emitting layer of the organic EL element include metal complexes of quinolinol derivatives such as Alq₃, various types of metal complexes, an anthracene derivative, a bisstyrylbenzene derivative, a pyrene derivative, an oxazole derivative, and a poly(p-phenylene vinylene) derivative. The light-emitting layer may also be composed of a host material and a dopant material. As the host material, an anthracene derivative is preferably used. Other examples of the host material include the above-listed light-emitting materials, and also heterocyclic compounds having an indole ring as a partial structure of a fused ring; heterocyclic compounds having a carbazole ring as a partial structure of a fused ring; and a carbazole derivative, a thiazole derivative, a benzimidazole derivative, and a polydialkylfluorene derivative. Examples of the dopant material include quinacridone, coumarin, rubrene, perylene, and derivatives thereof; a benzopyran derivative; a rhodamine derivative; and an aminostyryl derivative; and a green light-emitting material is particularly preferably used. These materials may be used singly to form a film as the layer, or two or more thereof may be mixed and used to form a single layer. Alternatively, these materials may be used to form a layered structure, and the layered structure may be composed of different layers each formed of a single kind of material, or composed of different layers each formed of a mixture of materials, or composed of a layer formed of a single kind of material and a layer formed of a mixture of materials.

A phosphorescent emitter can also be used as the light-emitting material. The phosphorescent emitter may be a complex of metal such as iridium or platinum. Examples thereof include a green phosphorescent emitter such as Ir(ppy)₃, a blue phosphorescent emitter such as FIrpic or FIr6, and a red phosphorescent emitter such as Btp₂Ir(acac). A green phosphorescent emitter is preferably used. As a host material in this case, a host material having hole-injecting/transporting capability may be used, including carbazole derivatives such as 4,4'-di(N-carbazolyl)biphenyl, TCTA, and mCP, and a host material having electron-transporting capability may also be used, including p-bis(triphenylsilyl)benzene, and 2,2',2"-(1,3,5-phenylene)-tris(1-phenyl-1H-benzimidazole).

In order to avoid concentration quenching, doping of the host material with the phosphorescent material is preferably performed by co-deposition in an amount within a range of 1 to 30 wt% based on the entire light-emitting layer.

As the light-emitting material, materials that emit delayed fluorescence can also be used, including CDCB derivatives such as PIC-TRZ, CC2TA, PXZ-TRZ, and 4CzIPN (see Non-Patent Literature 5, for example). The material or materials can be formed into a thin film by a known method, such as vapor deposition, spin coating or inkjet printing.

Furthermore, the organic EL element can include a hole-blocking layer. Examples of a material used for the hole-blocking layer include compounds exhibiting a hole-blocking effect, such as a phenanthroline derivative such as bathocuproine, a metal complex of a quinolinol derivative, such as aluminum(III)bis(2-methyl-8-quinolinato)-4-phenylphenolate (hereinafter abbreviated as BAlq), various types of rare-earth complexes, a triazole derivative, a triazine derivative, a pyrimidine derivative, an oxadiazole derivative, and a benzoazole derivative. These materials may also be used as the material for the electron-transporting layer. These materials may be used singly to form a film as the layer, or two or more thereof may be mixed and used to form a single layer. Alternatively, these materials may be used to form a layered structure, and the layered structure may be composed of different layers each formed of a single kind of material, or composed of different layers each formed of a mixture of materials, or composed of a layer formed of a single kind of material and a layer formed of a mixture of materials. The material or materials can be formed into a thin film by a known method, such as vapor deposition, spin coating or inkjet printing.

Examples of a material used for the electron-transporting layer of the organic EL element include metal complexes of quinolinol derivatives such as Alq₃ and BAlq, various types of metal complexes, a triazole derivative, a triazine derivative, a pyrimidine derivative, an oxadiazole derivative, a pyridine derivative, a benzimidazole derivative, a benzoazole derivative, a thiadiazole derivative, an anthracene derivative, a carbodiimide derivative, a quinoxaline derivative, a pyridoindole derivative, a phenanthroline derivative, and a silole derivative. These materials may be used singly to form a film as the layer, or two or more thereof may be mixed and used to form a single layer. Alternatively, these materials may be used to form a layered structure, and the layered structure may be composed of different layers each formed of a single kind of material, or composed of different layers each formed of a mixture of materials, or composed of a layer formed of a single kind of material and a layer formed of a mixture of materials. The material or materials can be formed into a thin film by a known method, such as vapor deposition, spin coating or inkjet printing.

Examples of a material used for the electron-injecting layer of the organic EL element include alkali metal salts such as lithium fluoride and cesium fluoride, alkaline earth metal salts such as magnesium fluoride, metal complexes of quinolinol derivatives such as lithium quinolinol, metal oxides such as aluminum oxide, and metals such as ytterbium (Yb), samarium (Sm), calcium (Ca), strontium (Sr), and cesium (Cs). However, when an electron-transporting layer and a cathode are suitably selected, the electron-injecting layer can be omitted.

Furthermore, a material obtained by n-doping a material normally used for the electron-injecting layer or the electron-transporting layer with a metal such as cesium can be used for the electron-injecting layer or the electron-transporting layer.

Examples of a material used for the cathode of the organic EL element of the present invention include a metal having a lower work function, such as aluminum; an alloy having an even lower work function, such as a magnesium-silver alloy, a magnesium-calcium alloy, a magnesium-indium alloy, and an aluminum-magnesium alloy; and ITO and IZO.

The pyrimidine compound represented by the general formula (1) above is preferably used for the capping layer of the organic EL element. These may be used singly to form a film as the layer, or any of these may be mixed with another material and used to form a single layer. Alternatively, these may be used to form a layered structure, and the layered structure may be composed of different layers each formed of a single kind of material, or composed of different layers each formed of a mixture of materials, or composed of a layer formed of a single kind of material and a layer formed of a mixture of materials. The material or materials for the layer can be formed into a thin film by a known method, such as vapor deposition, spin coating or inkjet printing.

The pyrimidine compound represented by the general formula (1) above has a refractive index of preferably 1.70 or more, and more preferably 1.85 or more, in a wavelength range of 450 to 700 nm.

The present invention has been described hereinabove on the basis of an organic EL element with a top emission structure. However, the present invention is not limited thereto, and can also be applied in a similar manner to an organic EL element with a bottom emission structure or an organic EL element with a dual emission structure, which emits light from both of the top side and the bottom side. In these cases, the electrode positioned on the side of the light-emitting element from which light is extracted to the outside is preferably transparent or translucent.

### Examples

Hereinafter, embodiments of the present invention will be described specifically by way of examples. However, the present invention is not limited to the examples below as long as it does not depart from the spirit thereof.

### Example 1

### <Synthesis of 4-(biphenyl-4-yl)-2,6-di{4-(benzoxazol-2-yl)phenyl}-pyrimidine (Compound (1-73)>

5.0 g of 4-(biphenyl-4-yl)-2,6-dichloropyrimidine, 11.7 g of 2-{4-(4,4,5,5,-tetramethyl-[1,3,2]dioxaborolan-2-yl)-phenyl}-benzoxazole, 0.4 g of tetrakis(triphenylphosphine)palladium(0), and 5.1 g of potassium carbonate were added to a reaction vessel, and the mixture was refluxed and stirred in toluene/EtOH/H₂O mixed solvent overnight. After the mixture was allowed to cool, MeOH was added thereto, and the precipitated solid, a crude product, was obtained by filtration. The obtained crude product was purified through crystallization from monochlorobenzene/acetone mixed solvent to obtain 8.1 g of white powder of 4-(biphenyl-4-yl)-2,6-di{4-(benzoxazol-2-yl)phenyl}-pyrimidine (Compound (1-73)) (yield: 78.9%).

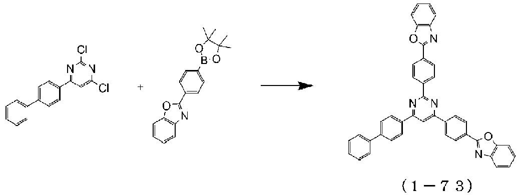

The structure of the obtained white powder was identified using NMR.

In ¹H-NMR (DMSO-d₆), the following signals of 26 hydrogens were detected, and thus it was seen that the obtained material is Compound (1-73).
δ (ppm) = 8.92 (2H), 8.81-8.79 (3H), 8.67 (2H), 8.45 (4H), 7.96 (2H), 7.90-7.82 (6H), 7.56-7.43 (7H).

### Example 2

### <Synthesis of 2,4,6-tri{4-(benzoxazol-2-yl)phenyl}-pyrimidine (Compound (1-92)>

4.3 g of 2,4,6-trichloropyrimidine, 25.0 g of 2-{4-(4,4,5,5,-tetramethyl-[1,3,2]dioxaborolan-2-yl)-phenyl}-benzoxazole, 0.8 g of tetrakis(triphenylphosphine)palladium(0), and 11.4 g of potassium carbonate were added to a reaction vessel, and the mixture was refluxed and stirred in toluene/EtOH/H₂O mixed solvent overnight. After the mixture was allowed to cool, the precipitated solid, a crude product, was obtained by filtration. The obtained crude product was purified through recrystallization from dichlorobenzene to obtain 7.3 g of white powder of 2,4,6-tri{4-(benzoxazol-2-yl)phenyl}-pyrimidine (Compound (1-92)) (yield: 47.1%).

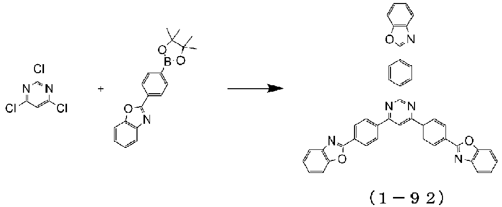

The structure of the obtained white powder was identified using NMR.

In ¹H-NMR (DMSO-d₆), the following signals of 25 hydrogens were detected, and thus it was seen that the obtained material is Compound (1-92).
δ (ppm) = 8.89 (2H), 8.82 (1H), 8.79 (4H), 8.42 (6H), 7.86-7.81 (6H), 7.51-7.40 (6H).

### Example 3

### <Synthesis of 4-(biphenyl-4-yl)-2,6-di {4-(benzothiazol-2-yl)phenyl} -pyrimidine (Compound (1-145)>

5.0 g of 4-(biphenyl-4-yl)-2,6-dichloropyrimidine, 12.3 g of 2-{4-(4,4,5,5,-tetramethyl-[1,3,2]dioxaborolan-2-yl)-phenyl]-benzothiazole, 0.4 g of tetrakis(triphenylphosphine)palladium(0), and 5.1 g of potassium carbonate were added to a reaction vessel, and the mixture was refluxed and stirred in toluene/EtOH/H₂O mixed solvent overnight. After the mixture was allowed to cool, MeOH was added thereto, and the precipitated solid, a crude product, was obtained by filtration. The obtained crude product was purified through crystallization from monochlorobenzene/acetone mixed solvent to obtain 10.3 g of white powder of 4-(biphenyl-4-yl)-2,6-di{4-(benzothiazol-2-yl)phenyl]-pyrimidine (Compound (1-145)) (yield: 95.1%).

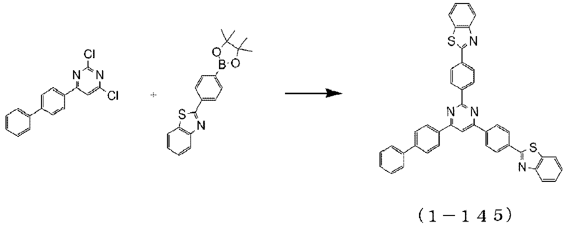

The structure of the obtained white powder was identified using NMR. In ¹H-NMR (DMSO-d₆), the following signals of 26 hydrogens were detected, and thus it was seen that the obtained material is Compound (1-145).
δ (ppm) = 8.87 (2H), 8.76-8.74 (3H), 8.66 (2H), 8.35 (4H), 8.21 (2H), 8.15 (2H), 7.96 (2H), 7.83 (2H), 7.61 (2H), 7.56-7.50 (4H), 7.45 (1H).

### Example 4

### <Synthesis of 2,4,6-tri{4-(benzothiazol-2-yl)phenyl}-pyrimidine (Compound (1-163)>

2.0 g of 2,4,6-trichloropyrimidine, 11.4 g of 2-{4-(4,4,5,5,-tetramethyl-[1,3,2]dioxaborolan-2-yl)-phenyl}-benzothiazole, 0.4 g of tetrakis(triphenylphosphine)palladium(0), and 6.0 g of potassium carbonate were added to a reaction vessel, and the mixture was refluxed and stirred in toluene/EtOH/H₂O mixed solvent overnight. After the mixture was allowed to cool, MeOH was added thereto, and the precipitated solid, a crude product, was obtained by filtration. The obtained crude product was purified through recrystallization from dichlorobenzene to obtain 6.7 g of white powder of 2,4,6-tri {4-(benzothiazol-2-yl)phenyl }-pyrimidine (Compound (1-163)) (yield: 86.8%).

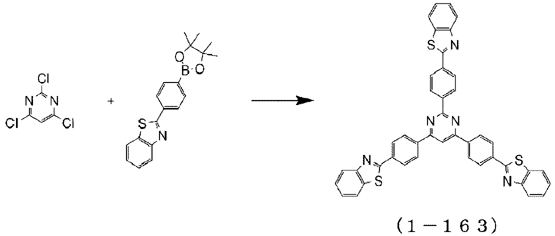

The structure of the obtained white powder was identified using NMR.

In ¹H-NMR (DMSO-d₆), the following signals of 25 hydrogens were detected, and thus it was seen that the obtained material is Compound (1-163).
δ (ppm) = 8.90 (2H), 8.84 (1H), 8.78 (4H), 8.38 (6H), 8.22 (3H), 8.16 (3H), 7.64-7.60 (3H), 7.55-7.51 (3H).

### Example 5

The melting point and the glass transition point (Tg) of each of the compounds obtained in Examples were measured using a high-sensitivity differential scanning calorimeter (DSC3100SA manufactured by Bruker AXS K.K.). Table 1 shows the measurement results.

**Table 1**

| | Compound | Melting point | Glass transition point |
|---|---|---|---|
| Ex. 1 | Compound (1-73) | 295°C | Not observed |
| Ex. 2 | Compound (1-92) | 369°C | 193°C |
| Ex. 3 | Compound (1-145) | 299°C | Not observed |
| Ex. 4 | Compound (1-163) | 355°C | Not observed |

It is seen from the results that the compounds obtained in Examples have a high melting point and have no glass transition point or a glass transition point of 100°C or higher. This indicates that these compounds are stable in the form of a thin film.

### Example 6

A vapor-deposited film with a thickness of 80 nm was formed on a silicon substrate by using each of the compounds obtained in Examples above, and the refractive index n and the extinction coefficient k at a wavelength of 450 nm and those at a wavelength of 750 nm were measured using a spectroscopic analyzer (F10-RT-UV manufactured by Filmetrics, INC.). For comparison, the measurement was also performed for Comparative Compound (2-1) having the structural formula below and Alq₃ (see Patent Literature 4, for example). Table 2 collectively shows the measurement results.

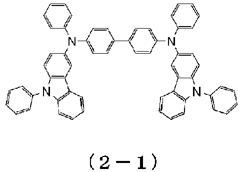

**Table 2**

| | Compound | Refractive index: n (λ: 450 nm) | Extinction coefficient: k (λ: 450 nm) | Refractive index: n (λ: 750 nm) | Extinction coefficient: k (λ: 750 nm) |
|---|---|---|---|---|---|
| Ex. 1 | Compound (1-73) | 2.12 | 0.00 | 1.89 | 0.00 |
| Ex. 2 | Compound (1-92) | 2.24 | 0.00 | 1.97 | 0.00 |
| Ex. 3 | Compound (1-145) | 2.18 | 0.00 | 1.94 | 0.00 |
| Ex. 4 | Compound (1-163) | 2.27 | 0.00 | 1.94 | 0.00 |
| Com.Ex. 1 | Alq3 | 1.88 | 0.00 | 1.73 | 0.00 |
| Com.Ex. 2 | Compound (2-1) | 1.84 | 0.00 | 1.77 | 0.00 |

As shown in Table 2, the refractive indices of the compounds of the present invention are higher than those of Alq₃ and Comparative Compound (2-1) in a wavelength range of 450 to 750 nm. Accordingly, it can be expected that using the compound of the present invention as a constituent material of the capping layer improves light extraction efficiency of the resulting organic EL element.

### Example 7

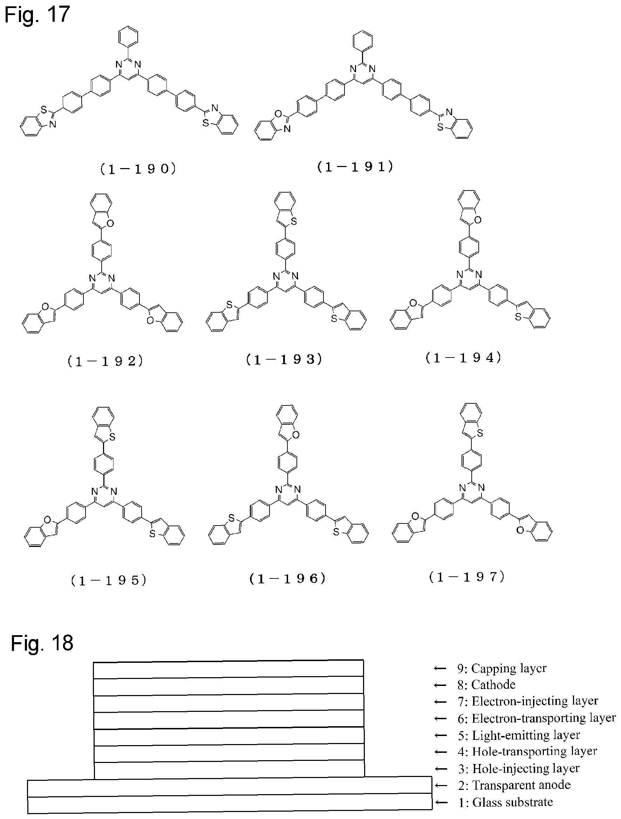

An organic EL element as shown in Fig. 18 was prepared by providing a glass substrate 1 having a reflecting ITO electrode formed thereon as a metal anode 2, and then vapor-depositing a hole-injecting layer 3, a hole-transporting layer 4, a light-emitting layer 5, an electron-transporting layer 6, an electron-injecting layer 7, a cathode 8, and a capping layer 9 in this order.

Specifically, the organic EL element was prepared in the following manner. A glass substrate 1 on which an ITO film with a thickness of 50 nm, a reflecting film made of silver alloy with a film thickness of 100 nm, and an ITO film with a thickness of 5 nm were formed in this order was ultrasonically cleaned in isopropyl alcohol for 20 minutes, and then dried for 10 minutes on a hot plate heated to 250°C. After that, UV/ozone treatment was performed for 2 minutes. Then, the glass substrate with ITO was set inside a vacuum vapor deposition machine, and the pressure was reduced to 0.001 Pa or less. Subsequently, an electron acceptor (Acceptor-1) having the structural formula below and Compound (3-1) having the structural formula below were vapor-deposited so as to coat the transparent anode 2 through binary vapor deposition at vapor deposition rates such that the ratio of the vapor deposition rate of Acceptor-1 to the vapor deposition rate of Compound (3-1) was 3:97, to thereby form a hole-injecting layer 3 with a thickness of 10 nm.

On the hole-injecting layer 3, a hole-transporting layer 4 (thickness of 140 nm) made of Compound (3-1) having the structural formula below was formed.

Compound (3-2) having the structural formula below and Compound (3-3) having the structural formula below were vapor-deposited on the hole-transporting layer 4 through binary vapor deposition at vapor deposition rates such that the ratio of the vapor deposition rate of (3-2) to the vapor deposition rate of (3-3) was 5:95, to thereby form a light-emitting layer 5 with a thickness of 20 nm.

Compound (3-4) having the structural formula below and Compound (3-5) having the structural formula below were vapor-deposited on the light-emitting layer 5 through binary vapor deposition at vapor deposition rates such that the ratio of the vapor deposition rate of (3-4) to the vapor deposition rate of (3-5) was 50:50, to thereby form an electron-transporting layer 6 with a thickness of 30 nm.

On the electron-transporting layer 6, an electron-injecting layer 7 (thickness: 1 nm) made of lithium fluoride was formed.

On the electron-injecting layer 7, a cathode 8 (thickness: 12 nm) made of magnesium-silver alloy was formed.

Finally, a capping layer 9 (thickness: 60 nm) made of Compound (1-73) of Example 1 was formed.

The resulting organic EL element was characterized in the atmosphere at normal temperature. Table 3 collectively shows the results of the determination of light emission properties when a DC voltage was applied to the organic EL element.

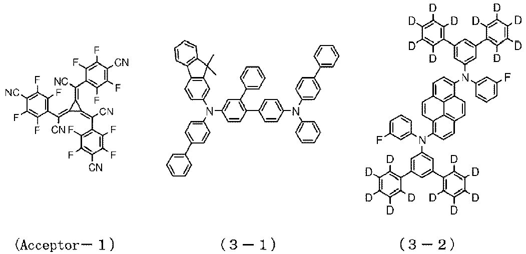

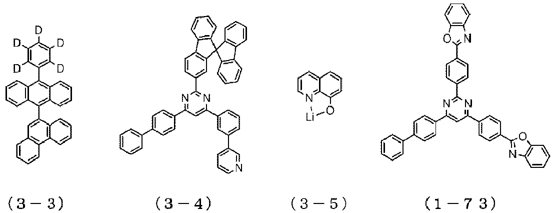

### Example 8

An organic EL element was prepared in the same manner as in Example 7, except that Compound (1-92) of Example 2 was used instead of Compound (1-73) of Example 1 to form a capping layer 9. The resulting organic EL element was characterized in the atmosphere at normal temperature. Table 3 collectively shows the results of the determination of light emission properties when a DC voltage was applied to the organic EL element.

### Example 9

An organic EL element was prepared in the same manner as in Example 7, except that Compound (1-145) of Example 3 was used instead of Compound (1-73) of Example 1 to form a capping layer 9. The resulting organic EL element was characterized in the atmosphere at normal temperature. Table 3 collectively shows the results of the determination of light emission properties when a DC voltage was applied to the organic EL element.

### Example 10

An organic EL element was prepared in the same manner as in Example 7, except that Compound (1-163) of Example 4 was used instead of Compound (1-73) of Example 1 to form a capping layer 9. The resulting organic EL element was characterized in the atmosphere at normal temperature. Table 3 collectively shows the results of the determination of light emission properties when a DC voltage was applied to the organic EL element.

### Comparative Example 1

For comparison, an organic EL element was prepared in the same manner as in Example 7, except that Alq₃ was used instead of Compound (1-73) of Example 1 to form a film with a thickness of 60 nm as the capping layer 9. The resulting organic EL element was characterized in the atmosphere at normal temperature. Table 3 collectively shows the results of the determination of light emission properties when a DC voltage was applied to the organic EL element.

### Comparative Example 2

For comparison, an organic EL element was prepared in the same manner as in Example 7, except that Compound (2-1) was used instead of Compound (1-73) of Example 1 to form a film with a thickness of 60 nm as the capping layer 9. The resulting organic EL element was characterized in the atmosphere at normal temperature. Table 3 collectively shows the results of the determination of light emission properties when a DC voltage was applied to the organic EL element.

The element lifespan of each of the organic EL elements prepared in Examples and Comparative Examples above were measured. Table 2 collectively shows the results. The element lifespan was defined as follows: the organic EL element was driven at a constant current of 10 mA/cm², and the time taken for the luminance to decay to 95% of the initial luminance (100%) was determined and defined as the element lifespan.

**Table 3**

| | Capping layer | Voltage [V] (@10 mA/cm²) | Luminance [cd/m²] (@10 mA/cm²) | Luminance efficiency [cd/A] (@10 mA/cm²) | Power efficiency [1m/W] (@ 10 mA/cm²) | Element lifespan (95% decay) |
|---|---|---|---|---|---|---|
| Ex. 7 | Compound (1-73) | 3.66 | 782 | 7.80 | 6.71 | 156 hours |
| Ex. 8 | Compound (1-92) | 3.65 | 793 | 7.92 | 6.82 | 167 hours |
| Ex. 9 | Compound (1-145) | 3.61 | 784 | 7.83 | 6.81 | 163 hours |
| Ex. 10 | Compound (1-163) | 3.66 | 800 | 8.00 | 6.88 | 164 hours |
| Com. Ex. 1 | Alq3 | 3.65 | 714 | 7.14 | 6.15 | 114 hours |
| Com. Ex. 2 | Compound (2-1) | 3.66 | 735 | 7.34 | 6.30 | 133 hours |

As shown in Table 3, while the elements of Comparative Examples 1 and 2 and those of Examples 7 to 10 had comparable driving voltages at a current density of 10 mA/cm², all of the elements of Examples achieved a significant improvement in the luminance, the luminance efficiency, the power efficiency, and the element lifespan as compared with those of Comparative Examples. This fact means that the compound represented by the general formula (1) of the present invention is a material suitable for use in a capping layer and can increase the refractive index of the capping layer, thereby significantly improving the light extraction efficiency of the organic EL element.

### Industrial Applicability

The compound of the present invention has a high refractive index to significantly improve the light extraction efficiency, and is stable in the form of a thin film, and thus it is a compound suitable for use in an organic EL element. Furthermore, the organic EL element produced by using the compound of the present invention has high efficiency. Furthermore, the compound of the present invention having no absorption in any of blue, green, and red wavelength regions is particularly preferably used to provide a clear and bright image with good color purity. Therefore, the present invention is expected to be applied to, for example, home electric appliances and lighting equipment.

### List of Reference Numerals

- 1: Glass substrate
- 2: Transparent anode
- 3: Hole-injecting layer
- 4: Hole-transporting layer
- 5: Light-emitting layer
- 6: Electron-transporting layer
- 7: Electron-injecting layer
- 8: Cathode
- 9: Capping layer

## Claims

1. A pyrimidine compound represented by a general formula (1) below: 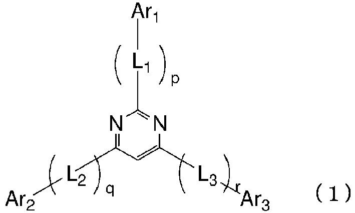
wherein Ar₁ to Ar₃ are the same or different, and each represent a substituted or unsubstituted aromatic hydrocarbon group, a substituted or unsubstituted aromatic heterocyclic group, or a substituted or unsubstituted fused polycyclic aromatic group,
L₁ to L₃ are the same or different, and each represent a single bond, a substituted or unsubstituted divalent aromatic hydrocarbon group, a substituted or unsubstituted divalent aromatic heterocyclic group, or a substituted or unsubstituted divalent fused polycyclic aromatic group,
p, q, and r are the same or different, and each represent an integer of 1 or 2, and
at least one of Ar₁ to Ar₃ is a substituted or unsubstituted benzoxazolyl group, a substituted or unsubstituted benzothiazolyl group, a substituted or unsubstituted benzofuranyl group, or a substituted or unsubstituted benzothienyl group.

2. The pyrimidine compound according to claim 1, wherein L₁ to L₃ in the general formula (1) above are the same or different, and each represent a single bond, a divalent group obtained by reducing two hydrogen atoms from a substituted or unsubstituted benzene, a divalent group obtained by reducing two hydrogen atoms from a substituted or unsubstituted biphenyl, or a divalent group obtained by reducing two hydrogen atoms from a substituted or unsubstituted naphthalene.

3. The pyrimidine compound according to claim 2, wherein L₁ to L₃ in the general formula (1) above are the same or different, and each represent a single bond, a divalent group obtained by reducing two hydrogen atoms from an unsubstituted benzene, a divalent group obtained by reducing two hydrogen atoms from an unsubstituted biphenyl, or a divalent group obtained by reducing two hydrogen atoms from an unsubstituted naphthalene.

4. The pyrimidine compound according to claim 3, wherein L₁ to L₃ in the general formula (1) above are the same or different, and each represent a single bond, an unsubstituted 1,4-phenylene group, an unsubstituted 4,4'-biphenylylene group, an unsubstituted 2,6-naphthylene group, or an unsubstituted 2,7-naphthylene group.

5. The pyrimidine compound according to claim 1, wherein p, q, and r in the general formula (1) above are 1.

6. The pyrimidine compound according to claim 1, wherein at least one of Ar₁ to Ar₃ in the general formula (1) above is an unsubstituted benzoxazolyl group, an unsubstituted benzothiazolyl group, an unsubstituted benzofuranyl group, or an unsubstituted benzothienyl group.

7. The pyrimidine compound according to claim 6, wherein at least one of Ar₁ to Ar₃ in the general formula (1) above is an unsubstituted 2-benzoxazolyl group, an unsubstituted 2-benzothiazolyl group, an unsubstituted 2-benzofuranyl group, or an unsubstituted 2-benzothienyl group.

8. The pyrimidine compound according to claim 7, wherein any two of Ar₁ to Ar₃ in the general formula (1) above are an unsubstituted 2-benzoxazolyl group, an unsubstituted 2-benzothiazolyl group, an unsubstituted 2-benzofuranyl group, or an unsubstituted 2-benzothienyl group.

9. The pyrimidine compound according to claim 7, wherein Ar₁ to Ar₃ in the general formula (1) above are an unsubstituted 2-benzoxazolyl group, an unsubstituted 2-benzothiazolyl group, an unsubstituted 2-benzofuranyl group, or an unsubstituted 2-benzothienyl group.

10. The pyrimidine compound according to claim 1, having a refractive index of 1.70 or more, in a wavelength range of 450 to 750 nm.

11. An organic electroluminescent element comprising, at least, an anode, a hole-transporting layer, a light-emitting layer, an electron-transporting layer, a cathode, and a capping layer arranged in this order,
wherein the capping layer comprises the pyrimidine compound according to any one of claims 1 to 10.

12. An organic electroluminescent element, wherein the capping layer is a mixed layer comprising two or more compounds or a stack of two or more layers comprising respective compounds different from each other, and at least one of the compounds is the pyrimidine compound according to any one of claims 1 to 10.

13. An electronic element comprising a pair of electrodes and at least one organic layer interposed therebetween,
wherein the organic layer comprises the pyrimidine compound according to any one of claims 1 to 10.

14. An electronic device comprising the electronic element according to claim 13.
